# FASCICULE DE BREVET EUROPEEN

(11) **EP 3 021 858 B1**
(45) Date de publication et mention de la délivrance du brevet: **05.05.2021**
(21) Numéro de dépôt: 14738881.3
(22) Date de dépôt: 24.06.2014
(51) Int. Cl.: A61K 35/742, A23L 33/135, A61P 29/02, A61P 43/00, A61K 39/07, A61P 19/02

(54) **SOUCHE CU1 POUR TRAITEMENT ET/OU PREVENTION D'UN RHUMATISME INFLAMMATOIRE CHRONIQUE**
STAMM CU1 ZUR BEHANDLUNG UND/ODER PRÄVENTION VON CHRONISCHEN ENTZÜNDUNGSRHEUMATISMUS
STRAIN CU1 FOR THE TREATMENT AND/OR PREVENTION OF CHRONIC INFLAMMATORY RHEUMATISM

(30) Priorité: 28.06.2013 FR 1356274
(43) Date de publication de la demande: 25.05.2016
(73) Titulaire: Lesaffre et Compagnie, 75001 Paris (FR); École Nationale Supérieure des Sciences Agronomiques de Bordeaux-Aquitaine, 33170 Gradignan (FR)
(72) Inventeur: TAILLADE, Patrick, F-40140 Azur (FR); URDACI, Marie, F-33170 Gradignan (FR)
(74) Mandataire: Plasseraud IP
(86) Numéro de dépôt international: PCT/FR2014/051561
(87) Numéro de publication internationale: WO 2014/207360

(56) Documents cités:
- EP-A1- 0 363 491
- WO-A1-2007/133188
- WO-A2-00/23471
- WO-A2-2005/019417
- WO-A2-2007/064741
- WO-A2-2009/026306
- FR-A1- 2 837 835
- US-A1- 2006 177 424

## Description

### DOMAINE DE L'INVENTION

La présente invention se situe dans le domaine des maladies inflammatoires. Elle concerne particulièrement l'utilisation d'une souche probiotique pour le traitement et/ou la prévention de maladies inflammatoires chroniques, en particulier de rhumatismes inflammatoires chroniques.

### ARRIÈRE PLAN TECHNIQUE

L'inflammation est un mécanisme de défense de l'organisme en réponse à une agression endogène ou exogène sur le corps humain. Des causes physiques, chimiques ou infectieuses peuvent être à l'origine d'agents exogènes de l'inflammation. Des causes métaboliques, des problèmes de vascularisation, ou une réponse immunitaire anormale peuvent être à l'origine d'agents endogènes de l'inflammation. En présence de l'un de ces agents, le mécanisme de l'inflammation va répondre par une série d'étapes successives :
- une réaction vasculo-exsudative faisant apparaitre une congestion active, un œdème inflammatoire, et une diapédèse leucocytaire,
- une réaction cellulaire consistant en la formation d'un granulome inflammatoire,
- une détersion, et
- une réparation et une cicatrisation de la zone lésée.

Toutefois, l'inflammation peut parfois être néfaste, due à la persistance d'un agent pathogène ou endogène, tel que l'auto-immunité lors de l'arthrite rhumatoïde. Les dommages causés à l'organisme vont alors continuer à se développer conduisant à une réponse immunitaire incontrôlée et à une inflammation chronique accompagnée d'une destruction des tissus tels que le tissu articulaire.

L'arthrite rhumatoïde est une maladie rhumatismale inflammatoire chronique évolutive, auto-immune qui touche de manière symétrique les articulations et qui se traduit par des douleurs, des gonflements et des raideurs des articulations au réveil. Elle est dite évolutive car dans la plupart des cas elle progresse par poussées inflammatoires entrecoupées de périodes de calme plus ou moins longues.

Elle peut aussi se manifester au niveau d'autres tissus et ces manifestations extra-articulaires peuvent alors mettre en danger la vie du patient.

L'arthrite rhumatoïde concerne environ 1% de la population avec une prépondérance chez la femme, 3 cas pour 1 chez les hommes, sachant que l'incidence augmente avec l'âge.

Les premiers symptômes apparaissent aux petites articulations des doigts ou des pieds. Avec le temps, l'inflammation chronique aboutit à la destruction de l'os et du cartilage et à une incapacité progressive. Si l'inflammation persiste, elle peut détruire le cartilage, le tissu osseux avoisinant, les tendons, et les ligaments péri-articulaires. Ainsi, la maladie a fréquemment pour conséquences de provoquer la déformation des articulations et une invalidité.

L'arthrite rhumatoïde se caractérise plus particulièrement par une inflammation de la membrane synoviale des articulations conduisant à une infiltration anormale de cellules inflammatoires dans le liquide synovial.

Dans un premier temps, il y a une prolifération anormale des vaisseaux sanguins ce qui permet un afflux de cellules pro-inflammatoires (lymphocytes dont les CD4, monocytes, polynucléaires, neutrophiles) dans la synoviale et l'espace intra-articulaire.

L'hyperplasie des fibroblastes synoviaux et l'infiltration de lymphocytes (T, CD4 et B) et de macrophages ont alors pour conséquence la formation d'un pannus synovial, ce qui se traduit par une inflammation de la membrane synoviale, appelée également synovite. Ces différentes cellules prolifèrent anormalement, envahissent l'os et le cartilage, produisent une quantité élevée de cytokines pro-inflammatoires et de métalloprotéinases, et déclenchent la formation et l'activation d'ostéoclastes. Les cytokines pro-inflammatoires particulièrement abondantes sont : TNF-α (*Tumor Necrosis Factor α*), IL-1, IL-6, IL-12 et IL-17, le terme IL désignant l'interleukine.

La destruction de l'articulation est secondaire à l'érosion de l'os et à la dégénérescence du cartilage. L'érosion est favorisée par la cytokine RANKL tandis que la chondrolyse est favorisée par l'enzyme métalloprotéinase. Ces deux molécules sont produites par des cellules qui composent la synoviale sous l'effet du TNF-α et de l'IL-1.

Les cellules vont ensuite essayer de contrer cette dégradation mais les ostéoclastes dont le rôle est de fabriquer de l'os, vont le fabriquer de manière anarchique produisant ainsi une déformation et une ossification des articulations.

Le traitement de l'arthrite rhumatoïde doit être initié dans les plus brefs délais à compter de la découverte de la maladie pour freiner le processus inflammatoire de façon à prévenir les lésions et les déformations articulaires.

Les traitements traditionnels consistent en l'administration d'anti-inflammatoires pour supprimer l'inflammation des articulations et la production des cytokines. Des médicaments immuno-modulateurs du système immunitaire sont également utilisés.

Les nouvelles thérapies actuelles portent notamment sur des agents bloquant du TNF-α ou de l'IL-1 tels que l'Etanercept, l'Infliximab, l'Adalimumab, le Certolizumab pégol.

Les réponses aux traitements varient selon les individus et l'état d'avancement de la maladie. De ce fait, la recherche de traitements alternatifs est importante pour offrir de nouvelles solutions aux patients résistants aux traitements existants.

De plus, le traitement de l'arthrite rhumatoïde a un coût élevé pour la société du fait des traitements anti-inflammatoires très onéreux et de l'invalidité du patient pouvant en résulter. En effet, les traitements pour les maladies inflammatoires sont à prendre sur le long terme pour obtenir des résultats, c'est-à-dire pour stopper la progression de la maladie, les poussées inflammatoires, stabiliser et maintenir la maladie dans un état de rémission.

De même, l'utilisation prolongée d'anti-inflammatoires par le patient peut avoir des effets indésirables tels qu'une prise de poids, des troubles gastro-intestinaux, et/ou des risques accrus d'ostéoporose et d'hypertension.

A ce titre, les probiotiques pourraient constituer une alternative intéressante.

Le terme « probiotique » est utilisé pour définir des microorganismes vivants qui, lorsqu'ils sont intégrés en quantité suffisante, exercent un effet positif sur la santé, le confort et le bien-être, au-delà des effets nutritionnels traditionnels.

Le document WO2007/133188 décrit l'utilisation d'une composition bactérienne antifongique comprenant au moins une bactérie choisie parmi *Bacillus subtilis, Bacillus coagulans* et *Enterococcus faecalis,* pour stimuler une réponse antifongique et pour le traitement de nombreuses pathologies très diverses, allant de l'autisme, aux infections vaginales, à l'endométriose, et parmi lesquelles est citée l'arthrite rhumatoïde. Ce document ne décrit pas de souches particulières de bactéries pouvant être utilisées dans la composition antifongique.

Le document WO 2007/064741 décrit l'utilisation d'une souche de *Bacillus subtilis,* PB6, pour son utilisation dans le traitement et/ou la prévention des diarrhées dues à l'antibiothérapies, des infections digestives dues à Clostridium difficile, des maladies gastro-intestinales et le syndrome de l'intestin irritable.

Le document WO 2005/019417 décrit une co-culture bactérienne comprenant une première souche de *Bacillus licheniformis* PA et une deuxième souche de *Bacillus subtilis* HE et pouvant être utilisée comme probiotique dans le traitement des maladies gastro-intestinales et d'autres maladies comme l'arthrite rhumatoïde.

Le document WO2009/026306 décrit par exemple une composition comprenant trois souches de bactéries, à savoir *Bacillus subtilis, Bacillus coagulans* et *Enterococcus faecium* ou *faecalis,* pour la prévention et/ou le traitement de nombreuses pathologies très diverses, allant de l'autisme, aux infections vaginales, à l'endométriose, et parmi lesquelles est citée l'arthrite rhumatoïde. Ce document ne décrit pas de souches particulières de bactéries pouvant être utilisées dans la composition.

Or, il est connu que l'effet probiotique d'une souche donnée, qu'il s'agisse d'une souche de levure ou d'une souche de bactérie est spécifique de cette souche, et non du genre ni même de l'espèce considérée.

Aussi, il existe toujours un besoin de trouver de nouveaux traitements pour la prévention et/ou le traitement de maladies inflammatoires chroniques, alternatifs à ceux existant, moins coûteux et présentant moins d'effets secondaires indésirables.

### DESCRIPTION DETAILLEE

La présente invention a ainsi pour objet une souche probiotique bactérienne pour son utilisation dans le traitement et/ou la prévention d'un rhumatisme inflammatoire chronique. Les inventeurs ont en effet découvert de manière surprenante que la souche *Bacillus subtilis* CU1 a un effet bénéfique dans le traitement et/ou la prévention de certaines maladies inflammatoires chroniques.

La souche *Bacillus subtilis* CU1 a été déposée sous le numéro I-2745 le 25 octobre 2001 à la CNCM, Collection Nationale de Cultures de Microorganismes de l'Institut Pasteur, au 25 rue du Docteur Roux, F-75725 Paris Cedex 15.

La souche *Bacillus subtilis* CU1 a été décrite pour la première fois dans le document FR 2 837 835. Ce document décrit la souche *Bacillus subtilis* CU1 comme un agent immuno-modulateur du système immunitaire et son utilisation pour la fabrication d'un vaccin vivant recombinant contre *H. pylori.*

Une « souche » désigne une population relativement homogène de cellules.

En particulier, une souche est obtenue à partir de l'isolement d'un clone, un clone étant une population de cellules obtenue à partir d'une seule cellule.

Les expressions « souche *Bacillus subtilis* CU1 », « souche probiotique CU1 », « souche *Bacillus subtilis* déposée à la CNCM sous le numéro I-2745 », et « souche CU1 » sont synonymes.

La présente invention a donc pour objet la souche *Bacillus subtilis* CU1 déposée à la CNCM sous le numéro I-2745 le 25 octobre 2001 pour son utilisation dans le traitement et/ou la prévention d'un rhumatisme inflammatoire chronique.

La présente invention a également pour objet les cellules obtenues par culture de la souche *Bacillus subtilis* CU1 déposée à la CNCM sous le numéro I-2745 le 25 octobre 2001, pour leur utilisation dans le traitement et/ou la prévention d'un rhumatisme inflammatoire chronique.

La présente invention a également pour objet une composition comprenant des cellules obtenues par culture de la souche *Bacillus subtilis* CU1 déposée à la CNCM sous le numéro I-2745 le 25 octobre 2001, pour son utilisation dans le traitement et/ou la prévention d'un rhumatisme inflammatoire chronique.

Les termes « cellules obtenues par culture de la souche *Bacillus subtilis* CU1 déposée à la CNCM sous le numéro I-2745 le 25 octobre 2001 », « biomasse CU1 », « cellules obtenues par culture de la souche CU1 » ou « cellules CU1 » sont ici synonymes.

La souche CU1 et les cellules CU1 obtenues par culture de ladite souche constituent un traitement alternatif pour la prévention et/ou le traitement d'un rhumatisme inflammatoire chronique, moins coûteux et ne présentant pas les effets indésirables des traitements classiques.

Les rhumatismes inflammatoires chroniques sont des maladies inflammatoires chroniques, comme le sont aussi le psoriasis, la pancréatite chronique, la maladie cœliaque, la thyroïdite de Hashimoto, la maladie de Crohn, les lupus érythémateux, la sclérodermie, la cirrhose biliaire primitive, la cholangite sclérosante, l'hépatite auto-immune et les vascularites.

On entend « par traitement d'une maladie » le fait de soigner une maladie afin d'en obtenir sa guérison, d'en soulager les symptômes ou les malaises, tel qu'en diminuant l'intensité inflammatoire, ou le fait de stabiliser une maladie afin de la maintenir dans un état de rémission.

On parle d'un état de rémission pour signifier l'intervalle entre deux poussées inflammatoires.

On entend par « prévention d'une maladie » le fait d'éviter l'apparition d'une maladie, par exemple chez un sujet à risque de développer la maladie, ou le fait de retarder une poussée inflammatoire de la maladie lorsque celle-ci est déjà présente.

Par « rhumatisme inflammatoire chronique », on désigne une maladie choisie dans le groupe comprenant l'arthrite rhumatoïde, le rhumatisme psoriasique, les spondylarthropathies, le lupus érythémateux systémique, le syndrome de Gougerot-Sjögren, la maladie de Behçet, la sclérodermie, l'arthrite juvénile idiopathique, et la polychondrite chronique atrophiante.

Dans un mode de réalisation préféré, la présente invention a pour objet la souche *Bacillus subtilis* CU1 ou les cellules obtenues par culture de ladite souche ou une composition comprenant des cellules obtenues par culture de ladite souche, pour son utilisation dans le traitement et/ou la prévention de l'arthrite rhumatoïde.

Les inventeurs ont en effet découvert de manière surprenante que l'utilisation de la souche probiotique *Bacillus subtilis* CU1, et en particulier de cellules obtenues par culture de ladite souche, permet de :
- diminuer la production de cytokines pro-inflammatoires telles que TNF-α, IL-1, IL-12, IL-17, comme démontré dans les exemples 1 et 2 ;
- stimuler la production des cytokines anti-inflammatoires telles que IL-10, comme démontré dans l'exemple 2 ;
- retarder et/ou diminuer les symptômes macroscopiques tels que la sévérité de l'inflammation ou le nombre d'articulations touchées lors de l'arthrite rhumatoïde, comme démontré dans l'exemple 1 ;
- diminuer l'infiltration des cellules immunitaires dans le liquide synovial, comme démontré dans l'exemple 1 ;
- réduire les lésions articulaires conduisant à une destruction du cartilage, de l'os et à une déformation des articulations, comme démontré dans l'exemple 1.

En effet, le TNF-α et les interleukines IL-1, IL-12 et IL-17 sont des cytokines pro-inflammatoires jouant un rôle primordial dans les maladies inflammatoires. Leur sécrétion excessive va engendrer une inflammation chronique ce qui, dans le cas de l'arthrite rhumatoïde, va provoquer des lésions irréversibles des articulations et leur déformation.

Par « symptômes macroscopiques » on entend les symptômes visibles à l'œil nu tels que la sévérité de l'inflammation, le nombre d'articulations touchées par l'inflammation, et la déformation des articulations, selon les critères de l'American College of Rheumatology (ACR) de 1987 révisés (cf. Arnett FC et al., Arthritis Rheum., 1988 ; 31:315-24).

Par « cellules immunitaires », on entend les macrophages, les lymphocytes, leucocytes circulaires polynucléaires et mononucléaires.

L'infiltration des cellules immunitaires dans l'articulation et plus particulièrement dans la membrane synoviale va produire un pannus synovial impliqué dans la destruction du cartilage et du tissu osseux avoisinant.

Les inventeurs ont ainsi découvert que l'administration de cellules CU1 avait pour effet de réduire et d'empêcher la formation d'un pannus synovial tel que décrit dans l'exemple 1. Par « lésions articulaires » on entend la destruction progressive du cartilage par perte des protéoglycanes de la matrice du cartilage, et de l'os des articulations ainsi que la déformation progressive desdites articulations. La déformation des articulations est due à une ossification anarchique de l'articulation par les ostéoclastes. Cette déformation peut avoir des conséquences importantes en conduisant à une perte d'autonomie et à une invalidité du patient.

L'utilisation de la souche *Bacillus subtilis* CU1 ou de cellules obtenues par culture de ladite souche ou d'une composition comprenant des cellules obtenues par culture de ladite souche dans le traitement et/ou la prévention d'un rhumatisme inflammatoire chronique peut être caractérisée en ce qu'elle induit une diminution de la production d'au moins une cytokine pro-inflammatoire et/ou une augmentation de la production d'au moins une cytokine anti-inflammatoire.

Une cytokine pro-inflammatoire dont la production est diminuée par la souche *Bacillus subtilis* CU1 ou par les cellules obtenues par culture de ladite souche ou par une composition comprenant des cellules obtenues par culture de ladite souche, est choisie dans le groupe comprenant le TNF-α, l'interleukine IL-1, l'interleukine IL-12 ou l'interleukine IL-17.

Une cytokine anti-inflammatoire dont la production est augmentée par la souche *Bacillus subtilis* CU1 ou par les cellules obtenues par culture de ladite souche ou par une composition comprenant des cellules obtenues par culture de ladite souche, est par exemple l'interleukine IL-10.

L'utilisation de la souche *Bacillus subtilis* CU1 ou de cellules obtenues par culture de ladite souche ou d'une composition comprenant des cellules obtenues par culture de ladite souche dans le traitement et/ou la prévention d'un rhumatisme inflammatoire chronique peut être caractérisée en ce qu'elle induit une diminution de la production d'au moins deux cytokines pro-inflammatoires, de préférence au moins trois cytokines pro-inflammatoires et/ou une augmentation de la production d'au moins une cytokine anti-inflammatoire, comme l'interleukine IL-10.

Préférablement, l'utilisation de la souche *Bacillus subtilis* CU1 ou de cellules obtenues par culture de ladite souche ou d'une composition comprenant des cellules obtenues par culture de ladite souche dans le traitement et/ou la prévention d'un rhumatisme inflammatoire chronique peut être caractérisée en ce qu'elle induit une diminution de la production de TNF-α, interleukine IL-1, interleukine IL-12 et interleukine IL-17 et une augmentation de la production de l'interleukine IL-10.

L'utilisation de la souche *Bacillus subtilis* CU1 ou de cellules obtenues par culture de ladite souche ou d'une composition comprenant des cellules obtenues par culture de ladite souche dans le traitement et/ou la prévention d'un rhumatisme inflammatoire chronique peut être caractérisée en ce qu'elle est destinée à un usage humain et/ou vétérinaire.

Un usage vétérinaire concerne à la fois les animaux d'élevage tels que le porc, la vache, le mouton, ou la chèvre, mais également les animaux domestiques tels que le chat, le chien, le cheval ou le lapin.

En particulier, l'utilisation de la souche *Bacillus subtilis* CU1 ou de cellules obtenues par culture de ladite souche ou d'une composition comprenant des cellules obtenues par culture de ladite souche dans le traitement et/ou la prévention d'un rhumatisme inflammatoire chronique est caractérisée en ce qu'elle est destinée à un usage humain.

Les cellules CU1 sont donc obtenues par culture de la souche *Bacillus subtilis* CU1 dans un milieu de culture, selon des procédés bien connus de l'homme du métier, comme par exemple décrit dans le livre Biotechnologie, 5e édition, R. Scriban, Edition Tec. & Doc., 1999, ISBN : 2-7430-0309-X.

Typiquement, un procédé de production de cellules CU1 par culture de la souche CU1 comprend les étapes de :
- ensemencement d'un milieu de culture avec un inoculum de la souche CU1,
- culture en condition aérobie, pour obtenir la multiplication des cellules,
- séparation de la biomasse de son milieu de culture, pour obtenir des cellules CU1.

Les cellules CU1 ainsi obtenues sont majoritairement sous forme végétative.

La « forme végétative » d'une bactérie désigne la forme d'une bactérie placée dans des conditions favorables.

L'expression «majoritairement sous forme végétative» signifie qu'au moins 70% des cellules sont sous forme végétative, de préférence au moins 80 %, et plus préférentiellement au moins 90%.

Un exemple de conditions favorables est un milieu de culture non limitant à une température et un pH favorables à la multiplication bactérienne.

Un milieu de culture non limitant contient tous les éléments nutritifs nécessaires à la multiplication des cellules.

Le procédé de production de cellules CU1 peut également comprendre une étape intermédiaire, entre l'étape de culture en condition aérobie et l'étape de séparation de la biomasse de son milieu de culture, de mise en condition défavorable des cellules. Les cellules CU1 obtenues à l'issue du procédé sont alors majoritairement sous forme sporulée. La « forme sporulée » d'une bactérie désigne la forme d'une bactérie placée dans des conditions défavorables.

L'expression « majoritairement sous forme sporulée » signifie qu'au moins 70% des cellules sont sous forme sporulée, de préférence au moins 80%, et plus préférentiellement au moins 90%.

La forme sporulée est ainsi une forme de résistance qui permet aux cellules de résister à un milieu difficile tel qu'un manque de nutriments, c'est-à-dire un milieu nutritif limitant, un stress hydrique, une variation importante du pH ou de la température, ou encore à la traversée du tube digestif.

La mise en condition défavorable des cellules CU1 est par exemple obtenue par : non renouvellement du milieu de culture des bactéries, arrêt de l'alimentation en milieu de culture, utilisation d'un milieu de culture limitant, changement de température, changement de pH, une maitrise de l'aération et/ou de l'agitation de sorte à maintenir une pression d'O₂ supérieure à 20 % dans le milieu de culture et une pression en CO₂ inférieure à 1,5 % dans les gaz sortants ou leur combinaison.

Il est également possible d'obtenir des cellules CU1 majoritairement sous forme sporulée en ajoutant de l'acide glutamique dans le milieu de culture en une quantité de l'ordre de 0,75g/L.

La présente invention a ainsi également pour objet des cellules obtenues par culture de la souche CU1 ou une composition comprenant lesdites cellules, pour leur utilisation dans le traitement et/ou la prévention d'un rhumatisme inflammatoire chronique, caractérisées en ce que les cellules sont sous forme sporulée et/ou sous forme végétative.

Dans un mode de réalisation préféré, les cellules CU1 sont majoritairement sous forme sporulée.

Le procédé de production de cellules CU1 peut également comprendre une étape ultérieure de séchage des cellules CU1, pour obtenir des cellules CU1 sous forme sèche.

Le séchage est par exemple un séchage par lyophilisation, un séchage sur lit fluidisé ou un séchage par atomisation.

La présente invention a ainsi pour objet des cellules obtenues par culture de la souche CU1 ou une composition comprenant lesdites cellules, pour leur utilisation dans le traitement et/ou la prévention d'un rhumatisme inflammatoire chronique, caractérisées en ce que lesdites cellules sont sous forme sèche.

Des « cellules sous forme sèche » signifie que la biomasse obtenue à l'issue du procédé de production des cellules CU1 comprend plus de 90% de matières sèches, de préférence plus de 95% de matière sèche.

Le dosage journalier dépend à la fois du type d'animal, de son poids, du mode d'administration et du type de traitement curatif ou préventif.

Les cellules ou la composition telles que définies ci-dessus peuvent faire l'objet d'une utilisation quotidienne des cellules CU1 dans une quantité de 1.10⁸ UFC à 1. 10¹¹ UFC lorsqu'elle est destinée à un usage humain, de préférence 5.10⁸ UFC à 1.10¹⁰ UFC.

Les cellules ou la composition telles que définies ci-dessus peuvent faire l'objet d'une utilisation quotidienne des cellules CU1 dans une quantité de 1.10⁵ UFC à 1.10¹¹ UFC lorsqu'elle est destinée à un usage vétérinaire, de préférence 1.10⁷ UFC à 1.10¹⁰ UFC.

Le terme UFC désigne une Unité Formant Colonie.

La présente invention a par ailleurs pour objet une composition telle que définie ci-dessus, pour son utilisation dans le traitement et/ou la prévention d'un rhumatisme inflammatoire chronique, caractérisée en ce que la composition est une composition alimentaire, un complément alimentaire ou une composition pharmaceutique.

La composition alimentaire désigne n'importe quel aliment, boisson, ou confiserie.

La composition alimentaire peut par exemple être une boisson, une barre de céréales, un chewing-gum, un produit laitier, tel qu'un produit laitier fermenté.

On entend par « complément alimentaire » une denrée alimentaire dont le but est de compléter le régime alimentaire normal et qui constitue une source concentrée de nutriments ou d'autres substances ayant un effet nutritionnel ou physiologique, seuls ou combinés.

Un complément alimentaire est commercialisé sous forme de dose, par exemple les formes de présentation telles que gélule, pastille, comprimé, pilule et autres formes similaires, sachet de poudre, ampoule de liquide, flacon muni d'un compte-goutte et autres formes analogues de préparations liquides ou en poudres destinées à être prises en unités mesurées de faible quantité.

Une composition pharmaceutique comprend en outre un véhicule pharmaceutiquement acceptable.

La présente invention a également pour objet la composition telle que définie ci-dessus, pour son utilisation dans le traitement et/ou la prévention d'un rhumatisme inflammatoire chronique, caractérisée en ce que la composition est destinée à une administration par voie orale ou par voie topique, de préférence par voie orale.

La présente invention a ainsi pour objet la composition telle que définie ci-dessus, pour son utilisation dans le traitement et/ou la prévention d'un rhumatisme inflammatoire chronique, caractérisée en ce que la composition est sous forme de gélule, pastille, pilule, comprimé, capsule, poudre, suspension, solution liquide, granule, gel ou crème.

La présente invention a également pour objet une composition telle que définie ci-dessus pour son utilisation dans le traitement et/ou la prévention d'un rhumatisme inflammatoire chronique, caractérisée en ce qu'elle comprend en outre un autre principe actif.

L'autre principe actif est de préférence choisi dans le groupe des principes actifs ayant une activité anti-inflammatoire, une activité immunomodulatrice du système immunitaire et/ou une activité antalgique.

Avantageusement, l'autre principe actif n'est pas une souche de *Bacillus subtilis,* de préférence n'est pas une souche de bactérie, plus préférentiellement n'est pas une souche probiotique.

Préférablement, la composition selon l'invention contient des cellules obtenues par culture de la souche CU1, à l'exclusion de toute autre souche de bactérie.

Préférablement, la composition selon l'invention contient des cellules obtenues par culture de la souche CU1, à l'exclusion de toute autre souche de levure ou de bactérie.

Egalement préférablement, la composition selon l'invention contient des cellules obtenues par culture de la souche CU1 comme seul principe actif.

Est également décrite ici une méthode de traitement et/ou de prévention d'une maladie inflammatoire chronique chez un patient comprenant l'administration audit patient d'une quantité efficace de cellules obtenues par culture de la souche CU1 ou d'une composition comprenant lesdites cellules.

La méthode de traitement et/ou de prévention d'une maladie inflammatoire chronique chez un patient comprenant l'administration audit patient d'une quantité efficace de cellules obtenues par culture de la souche CU1 ou d'une composition comprenant lesdites cellules, est caractérisée en ce que ladite maladie inflammatoire chronique est un rhumatisme inflammatoire chronique.

Préférablement, la présente invention a pour objet une méthode de traitement et/ou de prévention de l'arthrite rhumatoïde chez un patient comprenant l'administration audit patient d'une quantité efficace de cellules obtenues par culture de la souche CU1 ou d'une composition comprenant lesdites cellules.

La présente invention va maintenant être illustrée à l'aide des exemples et des figures qui suivent, qui sont donnés à titre d'illustration, et ne sont nullement limitatifs.

### BREVE DESCRIPTION DES FIGURES

Figure 1 : Scores cliniques cumulés de tous les rats par groupe sur les 50 jours de l'étude selon l'essai n°1.
Figure 2 : Evolution des scores cliniques moyens des rats des groupes G2 et G4 au cours de l'essai n°2 et à compter de la 1^{ère} immunisation à J+7.
Figure 3 : Scores cliniques cumulés de tous les rats par groupe, sur les 43 jours de l'étude selon l'essai n°2.
Figure 4 : Scores cliniques cumulés de tous les rats par groupe, sur les 50 jours de l'étude selon l'essai n°3.
Figure 5 : Dosage du TNF-alpha (en pg/ml) dans le sérum des rats de chaque groupe selon l'essai n°2
Figure 6 : Dosage du TNF-alpha (en pg/ml) dans le sérum des rats de chaque groupe selon l'essai n°3
Figure 7 : Dosage de l'interleukine Il-17 (en pg/ml) dans le sérum des rats de chaque groupe selon l'essai n°2
Figure 8 : Dosage de l'interleukine Il-17 (en pg/ml) dans le sérum des rats de chaque groupe selon l'essai n°3
Figure 9 : Dosage du TNF- α (en pg/ml) dans les surnageants de chaque groupe, récupérés suite à la stimulation des PBMC humain par du *Mycobacterium butyricum.*
Figure 10 : Dosage de l'interleukine 1 (en pg/ml) dans les surnageants de chaque groupe, récupérés suite à la stimulation des PBMC humain par du *Mycobacterium butyricum.*
Figure 11 : Dosage de l'interleukine 12 (en pg/ml) dans les surnageants de chaque groupe, récupérés suite à la stimulation des PBMC humain par du *Mycobacterium butyricum.*
Figure 12 : Dosage de l'interleukine 10 (en pg/ml) dans les surnageants de chaque groupe, récupérés suite à la stimulation des PBMC humain par du *Mycobacterium butyricum.*

### EXEMPLES

### Exemple 1 : Effets de Bacillus subtilis CU1 sur un modèle d'arthrite expérimentale induite chez le rat.

### Matériel et méthodes

### 1- Modèles animaux

Des rats Lewis femelles âgées de 6 semaines sont utilisés. L'arthrite est induite par administration d'une émulsion de *Mycobacterium butyricum* (Mb) en solution huileuse qui sert d'adjuvant.

Le modèle ainsi obtenu est relativement fidèle à la Polyarthrite humaine par les signes cliniques : symétrie dans les articulations impliquées, les articulations périphériques sont préférentiellement touchées, hyperplasie synoviale, infiltration des cellules inflammatoires, érosions articulaires marginales et une meilleure susceptibilité de la maladie chez les femelles. On retrouve également la cascade immuno-inflammatrice qui se produit in situ au niveau des sites inflammatoires.

Les *Mycobacterium butyricum* (Difco, USA) sont achetés sous une forme lyophilisée et inactivés par la chaleur. Ils sont ensuite émulsifiés à raison de 180 ng de Mb tués par millilitre d'une solution huileuse de vaseline, de Tween 80 et de tampon PBS.

### 2- Echantillon utilisé

Les cellules de *Bacillus subtilis* CU1 (ci-après CU1) à tester sont utilisées sous une forme sporulée et lyophilisée. Les cellules sont mises en suspension dans de l'eau physiologique pour les essais et des doses quotidiennes de 5 x 10⁹ bactéries/jour sont administrées aux rats par gavage.

### 3- Traitement/Protocole

Les essais se sont étalés sur une durée de 50 jours et ont été réalisés en triple : les deux premiers essais avec des lots de 5 rats et le troisième avec un lot de 12 rats traités avec CU1, les trois essais sont espacés dans le temps.

### Planning :

- J0 : les rats sont séparés en plusieurs lots :
   → Groupe 1 (G1) : témoins négatifs, rats sains ;
   → Groupe 2 (G2) : rats ayant une arthrite expérimentale sans traitement par CU1 ;
   → Groupe 3 (G3) : rats sains recevant la dose quotidienne de CU1 par gavage à compter de J0 et durant toute la durée de l'étude ;
   → Groupe 4 (G4) : rats ayant une arthrite expérimentale recevant la dose quotidienne de CU1 par gavage à compter de J0 et durant toute la durée de l'étude.
- J+7 : les rats du groupe G2 et G4 sont immunisés par une première injection intradermique de 300 µl de l'émulsion de Mb inactivés à la base de la queue, soit une dose de 54 ng d'une émulsion de Mb inactivés.
- J+14 : les rats du groupe G2 et G4 sont immunisés pour la seconde fois, dans les mêmes conditions.

### Scores cliniques de l'inflammation :

Les premiers signes cliniques apparaissent à J+23 chez les rats pour lesquels on a induit une arthrite. Les rats développent rapidement des signes d'arthrite au niveau des articulations périphériques (essentiellement les pattes postérieures) avec une forte réaction inflammatoire de la cavité synoviale se traduisant par une quasi-paralysie de l'articulation concernée et une érosion destructive du tissu osseux. Comme dans beaucoup de modèles pathologiques, l'animal subit une forte perte de poids.

Les symptômes inflammatoires sont « scorés » suivant un score clinique préétabli et résumé dans le tableau 1.

**Tableau 1**

| **Score clinique** | **Symptômes** |
|---|---|
| Score 0 | Aucune patte enflammée |
| Score 1 | Inflammation/paralysie d'une patte |
| Score 2 | Inflammation/paralysie de deux pattes |
| Score 3 | Inflammation/paralysie de trois pattes |
| Score 4 | Inflammation/paralysie de quatre pattes |

Les rats sont pesés et scorés quotidiennement.

### Protocole histologique

Les pattes sont fixées dans du formaldéhyde 4 % puis décalcifiées par électrophorèse dans un tampon de décalcification (Bayer Diagnostic, Puteau, France) pendant 3h. Ensuite, les pattes sont imprégnées de paraffine et des coupes histologiques sont obtenues avec un microtome.

Des sections de 5 mm sont colorées en utilisant les protocoles de routine.

Les coupes histologiques obtenues pour chacun des groupes sont colorées avec deux colorations différentes :
- Coloration à Hématoxyline - Éosine (HE), une coloration qui colore le collagène en rose pâle, les muscles en rose foncé, le cytoplasme acidophile en rouge, le cytoplasme basophile en violet, le noyau en bleu et les érythrocytes en rouge cerise ;
- Coloration au bleu d'aniline de Mallory ou triple coloration de Mallory (MA) mettant en évidence le tissu conjonctif : les fibrilles de collagène en bleu, les fibres musculaires en rouge, les fibrilles d'élastine en rose ou jaune.

Les coupes sont observées au microscope optique à un grossissement de x100 et x400.

### Protocoles de dosage des cytokines inflammatoires

Le dosage des cytokines est réalisé par la méthode ELISA en utilisant les kits anticorps et standards fournis par eBioscience (San Diego, Ca, USA) et les protocoles indiqués par le fournisseur (Rat IL-17A ELISA Ready-SET-Go ; Rat TNF alpha ELISA Ready-SET-Go).

### Résultats

### 1- Symptômes inflammatoires : score clinique de l'inflammation

Les rats sains recevant ou non la souche probiotique (G1 et G3) ne développent pas de symptômes inflammatoires, leurs scores cliniques sont donc nuls et ne sont pas représentés sur les courbes.

### Essai N°1 :

Les rats du groupe 2 développent une arthrite expérimentale dès le 19^{ème} jour suivant la 1ère immunisation (J+26) avec Mb. Leur score clinique évolue ensuite pour se situer en moyenne entre 3 et 4, 10 jours seulement après l'apparition des premiers signes cliniques (J+33). Ce score reste ensuite stable et élevé durant toute la durée de l'étude.

Au contraire, les rats du groupe 4 recevant les cellules CU1 ne développent pas de symptômes inflammatoires ou les développent plus tard que le groupe 2. De plus, les scores cliniques du groupe 4 sont nettement moins élevés ; et la sévérité et le nombre de pattes touchées restent inférieurs au groupe 2.

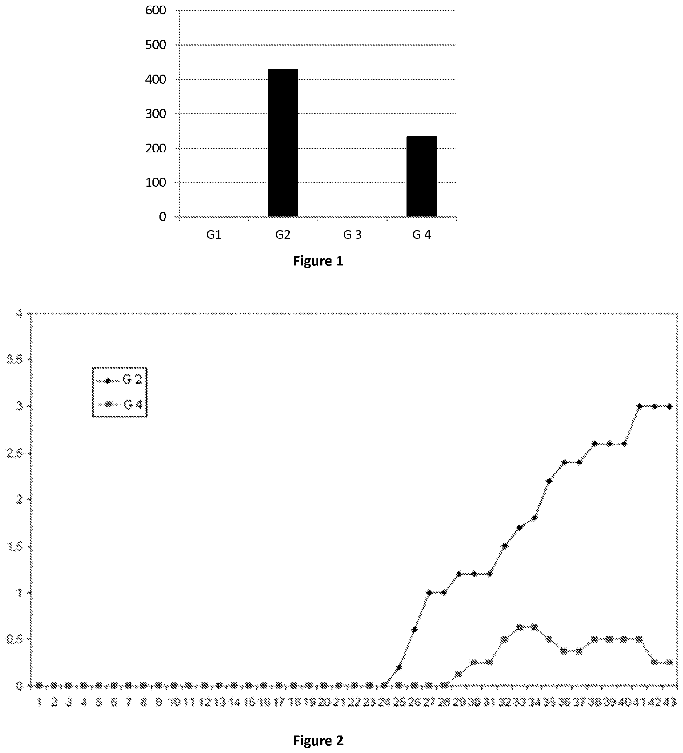

Selon la figure 1, on observe que les rats du groupe 2 atteignent un score cumulatif total de 426 contre 231 pour les rats du groupe 4 traités avec CU1. Ainsi, CU1 permet de diminuer de 46 % l'importance de signes pathologiques.

### Essai N°2 :

Les rats du groupe 2 développent une arthrite expérimentale dès le 25^{ème} jour suivant la 1^{ère} immunisation (J+32) avec Mb. Leur score clinique évolue ensuite rapidement pour s'établir à 3 en moyenne, 15 jours seulement après l'apparition des premiers signes cliniques (J+38). Ce score reste ensuite stable et élevé durant toute la durée de l'étude.

Les rats du groupe 4 recevant CU1 développent également des symptômes inflammatoires mais plus tardivement, le 28^{ème} jour (J+35) et de manière beaucoup plus faible. Au 33^{ème} jour après la 1^{ère} immunisation (J+40) les symptômes diminuent et restent stables ensuite jusqu'à la fin de l'étude.

Bien qu'enflammés, la sévérité et le nombre de pattes touchées restent inférieurs au groupe 2.

On peut donc observer une différence significative quant à l'apparition et à la sévérité des symptômes de l'inflammation (figure 2).

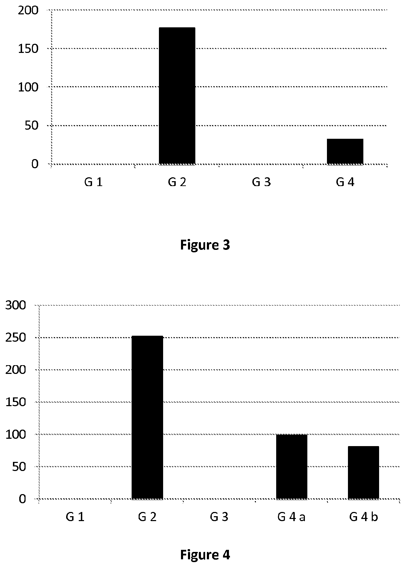

Selon la figure 3, on observe que les rats du groupe 2 atteignent un score cumulatif total de 176 contre 31 pour les rats du groupe 4 traités avec CU1. Ainsi, CU1 permet de diminuer de 82 % l'importance de signes pathologiques.

### Essai N°3 :

Deux lots de 6 rats sont utilisés dans le groupe 4, nommés a et b.

Selon la figure 4, on observe une différence significative entre les scores cumulés dans les groupes 2 et 4a/b. Les 6 rats du groupe 2 atteignent un score cumulatif total de 251 contre 98 pour les 6 rats traités avec CU1 (G4a) et 80 pour les 6 rats traités avec CU1 (G4b). Ainsi, CU1 permet de diminuer de 61 % (G4a) et de 68 % (G4b) l'importance de signes pathologiques.

### 2- Analyse visuelle des pattes arrière des rats

L'analyse visuelle est effectuée par l'observation visuelle de la présence d'inflammation sur les pattes arrière des rats des différents groupes.

Les pattes des rats du groupe 1 n'ayant subi aucun traitement ne présentent aucune inflammation. Les pattes des rats du groupe 2 présentent des inflammations importantes visibles. Les pattes des rats du groupe 4 ne présentent pas de signes d'inflammation ou très inférieurs au groupe 2.

### 3- Analyses histologiques des pattes arrière des rats

Les coupes du groupe 2 en coloration HE, présentent une déstructuration du tissu de l'articulation ainsi qu'une forte infiltration des cellules immunitaires au niveau de l'articulation par rapport aux groupes 1 et 3. L'observation des coupes du groupe 4 en coloration HE, permet de constater une moindre infiltration de cellules immunitaires dans l'articulation et que globalement le tissu des articulations est moins altéré.

Les coupes du groupe 2 en coloration MA, montrent une invasion de tissu conjonctif dans l'espace articulaire ainsi qu'une forte infiltration des leucocytes visible au grossissement x400, une perte de l'architecture articulaire et une érosion de l'os. L'observation des coupes du groupe 4 en coloration MA, permet de voir que certains animaux ne présentent pas ou presque pas de lésions au niveau des articulations et s'il y a des lésions, elles sont de moindre intensité par rapport aux lésions observées chez les animaux atteints du groupe 2. Il y a ainsi eu prévention de la formation du pannus, de l'inflammation de la synoviale, de la destruction du cartilage et de la déformation des articulations.

### 4- Dosage des cytokines inflammatoires dans le sérum des rats

### Dosage du TNF-α :

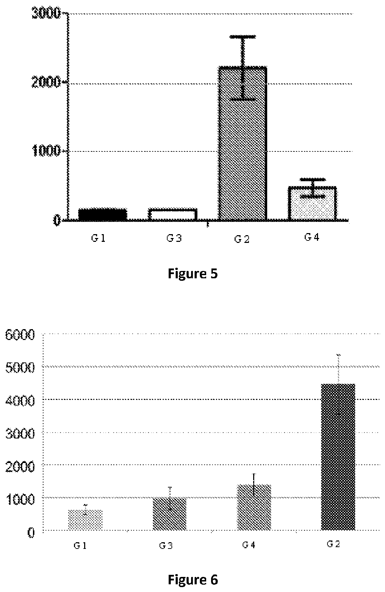

Les figures 5 et 6 représentent les résultats du dosage du TNF-α dans le sérum des rats de chaque groupe respectivement pour les essais 2 et 3. Le sérum de chaque animal est dosé en double puis on représente une moyenne globale de tous les animaux du groupe correspondant.

Le dosage du TNF-alpha est exprimé en picogramme par millilitre.

**Essai N°2** : Selon la figure 5, les groupes 1 et 3 présentent un taux faible de TNF- a, proche de 200 pg/ml. Le sérum des rats du groupe 2 présente une teneur en TNF-α de l'ordre de 2000 pg/ml. Le sérum des rats du groupe 4 présente une teneur en TNF-α de l'ordre de 500 pg/ml, soit 4 fois moins que le groupe 2.

**Essai N°3** : Selon la figure 6, le groupe 1 présente un taux en TNF-α d'environ 600 pg/ml et d'environ 1000 pg/ml pour le groupe 3. On observe également que le groupe 4 présente un taux peu élevé en TNF- α par rapport aux groupes 1 et 3, environ 1400 pg/ml, alors que le groupe 2 présente un taux élevé en TNF-α de l'ordre de 4400 pg/ml, soit trois fois plus que pour le groupe 4.

### Dosage de l'interleukine 11-17 :

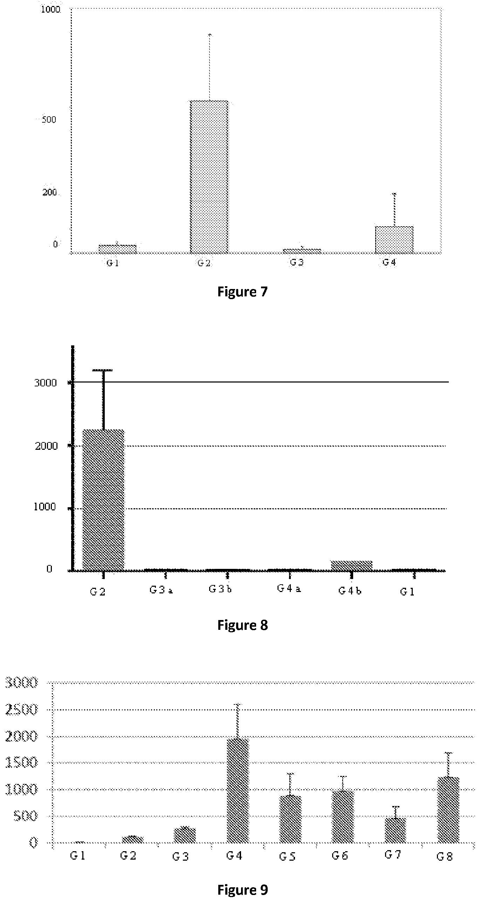

Les figures 7 et 8 représentent les résultats du dosage de l'Il-17 dans le sérum des rats de chaque groupe pour les essais 2 et 3.

Le dosage de l'Il-17 est exprimé en picogramme par millilitre.

**Essai N°2** : Selon la figure 7, les rats des groupes 1 et 3 ont un taux moyen en Il-17 faible, proche du seuil de détection du kit (50 pg/ml). On peut observer que le taux moyen en Il-17 du groupe 2 est élevé, environ 625 pg/ml, alors que le taux moyen en Il-17 du groupe 4 est faible, de l'ordre de 100 pg/ml.

**Essai N°3** : Selon la figure 8, les rats des groupes 1 et 3 ont un taux moyen en Il-17 faible, proche du seuil de détection du kit (50 pg/ml). On peut observer que le taux moyen en Il-17 du groupe 2 est élevé, supérieur à 2000 pg/ml, alors que le taux moyen en Il-17 dans le groupe 4 est faible dans les 2 lots, proche du seuil de détection du kit.

### Conclusion

Le modèle d'induction de l'arthrite utilisé a permis de démontrer que CU1 est capable de prévenir l'induction de la maladie ou de diminuer très significativement ses effets.

D'une part, on peut observer visuellement que les pattes des rats ne présentent pas d'inflammation ou présentent un score inflammatoire faible. L'administration de CU1 permet donc de diminuer l'importance desdits signes pathologiques.

D'autre part, l'analyse histologique des pattes de rats révèle que les rats arthritiques ayant eu un traitement avec CU1 (G4) présentent des lésions articulaires beaucoup moins prononcées, peu d'érosion de l'os, et une diminution ou disparition des infiltrations de cellules immunitaires dans les articulations par rapport aux rats n'ayant pas reçu le traitement avec CU1 (G2).

De plus, l'administration de CU1 aux rats arthritiques (G4) permet une diminution significative de la présence de cytokines pro-inflammatoires (TNF-α et IL17).

### Exemple 2 : Effets de Bacillus subtilis CU1 sur des cellules immunitaires humaines extraites à partir du sang de donneurs sains (PBMC) stimulées par Mycobacterium butyricum.

### Matériel et Méthode

### 1- Modèle cellulaire utilisé

Les PBMC (*Peripheral Blood Mononuclear Cell*) sont utilisées comme modèle dans cet exemple.

Le sang a été obtenu à partir de 4 donneurs volontaires sains, pré-testés pour l'absence d'infection VIH ou hépatites (EFS Aquitaine, Bordeaux Blood Bank). Les PBMC sont isolées par centrifugation dans un gradient de Ficoll™. Après lavage, les cellules sont remises en suspension à une concentration de 1 × 10⁶ PMBC/ ml dans le milieu RPMI complet.

Elles sont mises en contact avec du *Mycobacterium butyricum* (Mb) (Difco, USA) afin d'induire une réponse inflammatoire.

La solution mère de Mb est préparée par dilution du lyophilisat de l'exemple 1 dans du tampon PBS, à une concentration de 1 mg/ml dans du PBS. Puis, le nombre de cellules Mb dans la solution mère est compté au microscope en utilisant une cellule de Malassez afin que soit utilisé, lors des stimulations des PBMC, un rapport de 10 Mb par cellule immunitaire (MOI 10 :1). Le terme MOI (*Multiplicity Of Infection*) signifie la multiplicité d'infection.

### 2- Echantillon utilisé

Les cellules de *Bacillus subtilis* CU1 (ci-après CU1) à tester sont utilisées sous forme sporulée (CUsp) ou sous forme végétative (CUv). Une solution de travail à 2 x 10⁸ UFC/ ml dans de l'eau physiologique stérile est utilisée.

### 3- Stimulation des PBMC

Les expériences de stimulation sont réalisées en co-cultivant les PBMC et la souche CU1 selon un rapport de 10 bactéries par cellule immunitaire. Le lipopolysaccharide (LPS) a été utilisé comme contrôle positif de stimulation des PBMC pour la production de cytokines. Toutes les expériences sont effectuées en triplicata. Les surnageants de culture collectés après 18 h de contact sont centrifugés et conservés à -80°C jusqu'au dosage des cytokines (IL-1, TNF-α, IL-12 et IL-10).

### 4- Préparation des différents groupes de l'essai :

- Groupe 1 (G1) : présence uniquement des PBMC ;
- Groupe 2 (G2) : les PBMC sont mises en contact avec CU1 sous forme sporulée ;
- Groupe 3 (G3) : les PBMC sont mises en contact avec CU1 sous forme végétative ;
- Groupe 4 (G4) : les PBMC sont mises en contact avec Mb ;
- Groupe 5 (G5) : les PBMC sont mises en contact avec CU1 sous forme sporulée et Mb;
- Groupe 6 (G6) : les PBMC sont mises en contact avec CU1 sous forme végétative et Mb ;
- Groupe 7 (G7) : les PBMC sont préincubées 1 heure avec CU1 sous forme sporulée avant l'ajout de Mb ;
- Groupe 8 (G8) : les PBMC sont préincubées 1 heure avec CU1 sous forme végétative avant l'ajout de Mb.

Le dosage des cytokines a été réalisé par la méthode ELISA en utilisant les kits, anticorps et standards fournis par eBioscience (San Diego, Ca, USA) et les protocoles indiqués par le fournisseur.

### Résultats

### 1- Dosage du TNF-α

La figure 9 représente le dosage du TNF-α en pg/ml dans le surnageant récupéré à partir des PBMC pour chacun des groupes précités.

On observe un taux en TNF-α de l'ordre de 2000 pg/ml dans le groupe 4, alors que dans les groupes mis en contact avec CU1 (G5 et G6) la quantité de TNF-α est diminuée de moitié. De plus, on observe que la préincubation avec CU1 (G7 et G8) 1 heure avant l'ajout de Mb est plus efficace lorsque CU1 est sous forme sporulée (G7) ce qui permet d'abaisser le taux de TNF-α à environ 500 pg/ml (4 fois moins).

### 2- Dosage de l'interleukine IL-1

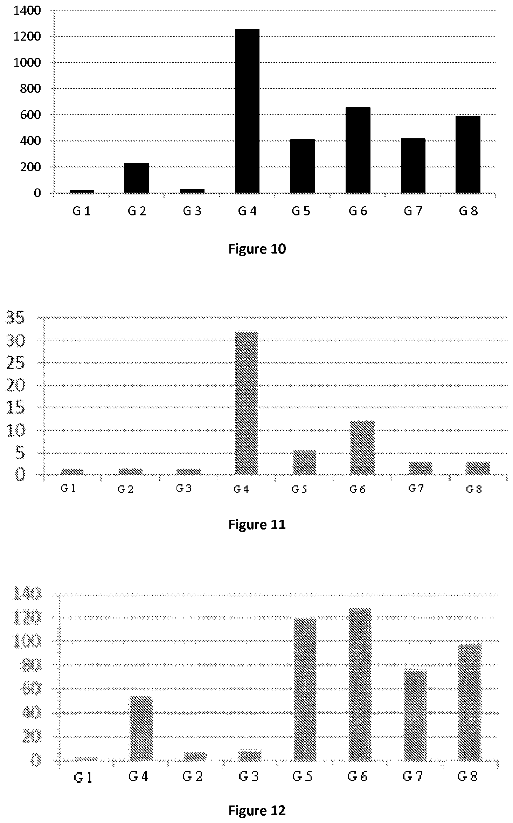

La figure 10 représente le dosage de l'IL-1 en pg/ml dans le surnageant récupéré à partir des PBMC pour chacun des groupes précités.

On observe un taux en IL-1 de l'ordre de 1250 pg/ml dans le groupe 4, alors que dans le groupe 5 le taux est de l'ordre de 400 pg/ml (3 fois moins), de même que dans le groupe 6. On n'a pas observé d'amélioration dans le cas des préincubations avec CU1 (G7 et G8) avant l'ajout de Mb.

### 3- Dosage de l'interleukine IL-12

La figure 11 représente le dosage de l'IL-12 en pg/ml dans le surnageant récupéré à partir des PBMC pour chacun des groupes précités.

On observe un taux en IL-12 de l'ordre de 32 pg/ml dans le groupe 4, alors que dans les groupes 5 et 6 mis en contact avec CU1 on observe une diminution importante d'IL-12, inférieure à 12 pg/ml. De plus, on observe que la préincubation avec CU1 (G7 et G8) avant l'ajout de Mb diminue davantage le taux d'IL-12 par rapport aux groupes 5 et 6.

### 4- Dosage de l'interleukine IL-10

La figure 12 représente le dosage de la cytokine anti-inflammatoire IL-10 en pg/ml dans le surnageant récupéré à partir des PBMC pour chacun des groupes précités.

On observe un taux en IL-10 de l'ordre de 55 pg/ml dans le groupe Mb, alors que dans les groupes 5 et 6 mis en contact avec CU1 on observe une augmentation de la production de cette cytokine anti-inflammatoire de l'ordre de 120 pg/ml (2 fois plus). De plus, on observe que la préincubation avec CU1 (G7 et G8) avant l'ajout de Mb ne semble pas efficace puisque les taux en IL-10 sont inférieurs (75 et 95 pg/ml) à ceux des groupes 5 et 6.

### Conclusion

Les résultats obtenus permettent de montrer de manière générale que l'administration de cellules *Bacillus subtilis* CU1 permet de diminuer la production des cytokines pro-inflammatoires et d'augmenter celle de la cytokine anti-inflammatoire IL-10.

## Revendications

1. Souche *Bacillus subtilis* CU1 déposée à la CNCM sous le numéro I-2745 le 25 octobre 2001, pour son utilisation dans le traitement et/ou la prévention d'un rhumatisme inflammatoire chronique.

2. Cellules obtenues par culture de la souche *Bacillus subtilis* CU1 déposée à la CNCM sous le numéro I-2745 le 25 octobre 2001, pour leur utilisation dans le traitement et/ou la prévention d'un rhumatisme inflammatoire chronique.

3. Composition pour son utilisation dans le traitement et/ou la prévention d'un rhumatisme inflammatoire chronique, la composition comprenant des cellules obtenues par culture de la souche *Bacillus subtilis* CU1 déposée à la CNCM sous le numéro I-2745 le 25 octobre 2001, à l'exclusion de toute autre souche de bactérie.

4. Composition pour son utilisation selon la revendication 3, **caractérisée en ce que** la composition est une composition alimentaire, un complément alimentaire ou une composition pharmaceutique.

5. Composition pour son utilisation selon la revendication 3 ou la revendication 4, **caractérisée en ce que** la composition est destinée à une administration par voie orale ou par voie topique.

6. Composition pour son utilisation selon l'une quelconque des revendications 3 à 5, **caractérisée en ce que** la composition est sous forme de gélule, pilule, pastille, comprimé, capsule, poudre, suspension, solution liquide, granule, gel ou crème.

7. Composition pour son utilisation selon l'une quelconque des revendications 3 à 6, **caractérisée en ce qu'**elle comprend en outre un autre principe actif.

8. Cellules pour leur utilisation selon la revendication 2, ou composition pour son utilisation selon l'une quelconque des revendications 3 à 7, **caractérisées en ce que** lesdites cellules sont sous forme sporulée et/ou sous forme végétative.

9. Cellules pour leur utilisation selon la revendication 2 ou la revendication 8, ou composition pour son utilisation selon l'une quelconque des revendications 3 à 8, **caractérisées en ce que** lesdites cellules sont sous forme sèche.

10. Souche pour son utilisation selon la revendication 1, ou cellules pour leur utilisation selon l'une quelconque des revendications 2, 8 et 9, ou composition pour son utilisation selon l'une quelconque des revendications 3 à 9, **caractérisées en ce que** ledit rhumatisme inflammatoire chronique est l'arthrite rhumatoïde.

## Patentansprüche

1. *Bacillus subtilis* CU1-Stamm, der am 25. Oktober 2001 bei der CNCM unter der Nummer I-2745 hinterlegt wurde, zur Verwendung bei der Behandlung und/oder Vorbeugung von chronisch entzündlichem Rheumatismus.

2. Zellen, die durch Kultivierung des *Bacillus subtilis*-Stamms CU1 gewonnen wurden, der am 25. Oktober 2001 bei der CNCM unter der Nummer I-2745 hinterlegt wurde, zur Verwendung bei der Behandlung und/oder Vorbeugung von chronisch entzündlichem Rheumatismus.

3. Zusammensetzung zur Verwendung bei der Behandlung und/oder Vorbeugung von chronisch entzündlichem Rheumatismus, wobei die Zusammensetzung Zellen umfasst, die durch Kultivierung des *Bacillus subtilis*-Stammes CU1 erhalten wurden, der am 25. Oktober 2001 unter der Nummer I-2745 bei der CNCM hinterlegt wurde, wobei alle anderen Bakterienstämme ausgeschlossen sind.

4. Zusammensetzung zur Verwendung nach Anspruch 3, **dadurch gekennzeichnet, dass** die Zusammensetzung eine Nahrungsmittelzusammensetzung, ein Nahrungsergänzungsmittel oder eine pharmazeutische Zusammensetzung ist.

5. Zusammensetzung zur Verwendung nach Anspruch 3 oder Anspruch 4, **dadurch gekennzeichnet, dass** die Zusammensetzung zur oralen oder topischen Verabreichung bestimmt ist.

6. Zusammensetzung zur Verwendung nach einem der Ansprüche 3 bis 5, **dadurch gekennzeichnet, dass** die Zusammensetzung in Form einer Gelkapsel, einer Pille, einer Lutschtablette, einer Tablette, einer Kapsel, eines Pulvers, einer Suspension, einer flüssigen Lösung, eines Granulats, eines Gels oder einer Creme vorliegt.

7. Zusammensetzung zur Verwendung nach einem der Ansprüche 3 bis 6, **dadurch gekennzeichnet, dass** sie ferner einen weiteren Wirkstoff enthält.

8. Zellen zur Verwendung nach Anspruch 2 oder Zusammensetzung zur Verwendung nach einem der Ansprüche 3 bis 7, **dadurch gekennzeichnet, dass** die Zellen in sporulierter und/oder vegetativer Form vorliegen.

9. Zellen zur Verwendung nach Anspruch 2 oder Anspruch 8 oder Zusammensetzung zur Verwendung nach einem der Ansprüche 3 bis 8, **dadurch gekennzeichnet, dass** die Zellen in trockener Form vorliegen.

10. Stamm zur Verwendung nach Anspruch 1 oder Zellen zur Verwendung nach einem der Ansprüche 2, 8 und 9 oder Zusammensetzung zur Verwendung nach einem der Ansprüche 3 bis 9, **dadurch gekennzeichnet, dass** der chronisch entzündliche Rheumatismus rheumatoide Arthritis ist.

## Claims

1. *Bacillus subtilis* strain CU1 deposited with the CNCM under number 1-2745 on 25 October 2001, for use thereof in the treatment and/or prevention of chronic inflammatory rheumatism.

2. Cells obtained by culture of the *Bacillus subtilis* strain CU1 deposited with the CNCM under number 1-2745 on 25 October 2001, for use thereof in the treatment and/or prevention of chronic inflammatory rheumatism.

3. Composition for use in the treatment and/or prevention of chronic inflammatory rheumatism, the composition comprising cells obtained by culture of the *Bacillus subtilis* strain CU1 deposited with the CNCM under number 1-2745 on 25 October 2001, to the exclusion of any other bacterial strain.

4. Composition for use according to claim 3, **characterised in that** the composition is a food composition, a food supplement or a pharmaceutical composition.

5. Composition for use according to claim 3 or claim 4, **characterised in that** the composition is intended for oral or topical administration.

6. Composition for use according to any one of claims 3 to 5, **characterised in that** the composition is in the form of a gel capsule, pill, lozenge, tablet, capsule, powder, suspension, liquid solution, granule, gel or cream.

7. Composition for use according to any one of claims 3 to 6, **characterised in that** it further comprises another active ingredient.

8. Cells for use according to claim 2, or composition for use according to any of claims 3 to 7, **characterised in that** said cells are in sporulated and/or vegetative form.

9. Cells for use according to claim 2 or claim 8, or composition for use according to any of claims 3 to 8, **characterised in that** said cells are in dry form.

10. Strain for use according to claim 1, or cells for use according to any one of claims 2, 8 and 9, or composition for use according to any one of claims 3 to 9, **characterised in that** said chronic inflammatory rheumatism is rheumatoid arthritis.
